# DEMANDE DE BREVET EUROPEEN

(11) **EP 0 880 946 A1**
(43) Date de publication de la demande: **02.12.1998**
(21) Numéro de dépôt: 97202698.3
(22) Date de dépôt: 02.09.1997
(51) Int. Cl.: A61F 2/06

(54) **Endoprothèse auto-expansible**

(30) Priorité: 27.05.1997 BE 9700461
(71) Demandeur: MEDICORP R&D BENELUX, 7000 Mons (BE)
(72) Inventeur: Frid, Noureddine, 1650 Beersel (BE)
(74) Mandataire: Bairiot-Delcoux, Mariette

(57) **Abrégé**

Une endoprothèse luminale comprenant une armature (1) formée de fils tressés.

L'armature (1) est auto-expansible, c'est-à-dire qu'après avoir été radialement comprimée pour la mise en place de l'endoprothèse, elle reprend d'elle-même son diamètre nominal. Le métal de l'armature a subi un traitement thermique provoquant une transition d'état, à une température proche de celle de l'organisme, ce qui lui confère une rigidité adéquate après sa mise en place dans un conduit anatomique.

## Description

L'invention concerne les endoprothèses luminales radialement expansibles et plus particulièrement les endoprothèses vasculaires et notamment, les "stents".

Depuis les travaux de C. DIDCOTT concernant la dilatation et le maintien de conduits anatomiques, le concept des endoprothèses dilatables a fait florès.

Une des percées les plus remarquables dans ce domaine concerne notamment la chirurgie vasculaire et cardiaque, notamment la réduction d'anévrismes et l'ouverture de sténoses.

Le taux de réussite général de ces méthodes a pour conséquence des exigences croissantes des praticiens tant en ce qui concerne la qualité des produits mis sur le marché que leur facilité de manipulation.

Des critères déterminants à cet égard sont le rapport élevé entre le diamètre de l'endoprothèse sous sa forme contractée et son diamètre nominal mais également la flexibilité de cette endoprothèse qui doit être à même, lors de l'insertion, de suivre des tracés sinueux, sans pour autant entraîner des plicatures (kinking).

Par ailleurs, lorsqu'elle est en place, une telle endoprothèse doit faire montre de caractéristiques mécaniques compatibles avec celles des vaisseaux traités et être à même de supporter les sollicitations à l'écrasement engendrées par la pression ambiante et la présence d'organes adjacents.

La recherche, dans les premiers temps, s'est surtout focalisée sur les vaisseaux sanguins de petit et moyen calibre mais il reste beaucoup à faire dans le domaine des vaisseaux de très faible diamètre et, à l'opposé, dans les conduits anatomiques de diamètre élevé.

Traiter des anévrismes thoraciques et abdominaux nécessite ainsi l'utilisation d'endoprothèses de gros diamètre: de l'ordre de 35 à 45 mm pour les anévrismes thoraciques et de l'ordre de 22 à 33 mm pour les anévrismes abdominaux.

Aucune des endoprothèses existant sur le marché dans cette gamme de diamètre ne répond à ce jour parfaitement aux attentes des praticiens, essentiellement parce qu'elles ne peuvent assumer leur rôle à long terme, qu'elles ne sont pas faciles à utiliser ou encore parce que les matériaux utilisés ne sont pas adaptés.

Les endoprothèses utilisées pour réparer les conduits anatomiques comprennent une armature rigide souvent pourvue d'un revêtement (ou "coating"). Les endoprothèses constituées simplement d'une armature portent le nom de "stent".

On trouve sur le marché, essentiellement deux types d'armatures (ou stents) en l'occurrence les armatures qui sont dilatées par ballons gonflables et des armatures autoexpansibles, qui comprennent des structures tressées ou non-tressées.

On connaît des endoprothèses qui sont mises en place puis dilatées à leur diamètre nominal par l'introduction d'un ballonnet gonflable.

Cette technique a pour inconvénient, notamment, l'interruption du flux sanguin et les dimensions de l'armature.

Les stents à ballons ne peuvent être utilisés que pour le traitement de lésions d'artères de petit calibre (au maximum 12 mm). La raison en est simple: pour dilater un stent de, par exemple, 3 mm de diamètre initial jusqu'à un diamètre de 8, 10 voire 12 mm, il est nécessaire d'utiliser une pression montant jusqu'à 5 à 10 atmosphères (comme signalé dans US-4,950,227).

Le ballon doit donc être extrêmement résistant, ce qui entraîne des problèmes de diamètre.

Par ailleurs il n'est pas possible de traiter de longues lésions par cette technique.

Il est à noter que l'intervention pour un anévrisme abdominal peut durer de 6 à 8 heures avec un abord chirurgical fémoral ou iliaque (à comparer avec une durée moyenne de 2 heures pour un traitement par voie chirurgicale directe).

Les stents autoexpansibles, quant à eux, n'ont pas besoin de ballons: ils sont généralement étirés en longueur et introduits, sous une forme diamétralement réduite, dans un applicateur consistant en un cathéter tubulaire muni d'un poussoir. L'ensemble est introduit notamment par voie fémorale ou iliaque jusqu'au site de largage, où l'endoprothèse est larguée.

Bien qu'ils présentent un certain nombre d'avantages, les modèles connus de stents autoexpansibles connaissent également une série de limitations, longtemps considérées comme incontournables. Leur diamètre ne dépasse généralement pas 25 mm.

Les stents tressés avec des alliages en Cobalt-Nickel-Chrome (ELGILOY® ou PHYNOX®) permettent néanmoins quant à eux d'obtenir des diamètres variant de 2 mm à 45 ou même 50 mm.

L'endoprothèse, soumise initialement à une élongation avec rétrécissement de diamètre, reprend d'elle-même son diamètre nominal lors du largage.

Les premières endoprothèses tressées de ce type ont été réalisées par C. DIDCOTT:

On connaît par FR-1.602.513 des endoprothèses dotées d'une armature rigide formée par tissage en tressage de fils métalliques.

Ce document décrit des tresses ayant un angle de croisement α entre les fils de deux nappes différentes compris entre 45 et 90°. Il va de soi qu'une tresse, du strict point de vue mécanique, résiste à l'écrasement d'autant moins bien que les fils tressés qui la constituent s'écartent d'une structure quasi annulaire, en l'occurrence une spirale au pas très faible, correspondant à un angle aussi proche que possible de 90° par rapport à l'axe de la tresse (comme décrit dans FR-2.333.487). Plus cet angle est faible, moins bien la tresse résiste à l'écrasement.

Le brevet US-5,061,275 décrit une endoprothèse à armature tressée où l'angle de croisement α est obtus. Dans ce cas, le coefficient d'allongement de la prothèse est élevé, ce qui entraîne des problèmes lors de la mise en place. (On définit par coefficient d'allongement le rapport de l'extension axiale d'une telle prothèse sous sa forme contrainte -donc à diamètre réduit- et sous sa forme non contrainte, à son diamètre nominal).

Le largage de ce type d'endoprothèse demande ainsi une longue pratique, le repérage étant difficile (l'endoprothèse subit un raccourcissement important au moment de sa libération). L'endoprothèse occupe une grande longueur dans l'introducteur, ce qui crée des frictions et réduit la maniabilité.

Les chercheurs qui se sont attelés à résoudre les problèmes liés à l'emploi de prothèses autoexpansibles à effet mécanique se sont heurtés à des questions d'angle, d'épaisseur et de composition des fils sans parvenir à obtenir une prothèse réunissant tous les critères de qualité: on ne parvient pas à obtenir une prothèse combinant un faible angle de croisement et une bonne résistance à l'écrasement.

On notera également que pour un même angle α = 85°, une tresse de 32 fils présente une résistance à la pression radiale de 50% plus élevée qu'une tresse de 24 fils de diamètre identique, ce qui montre qu'une telle structure répond à des relations relativement complexes.

La demande EP-A-0 740 928 décrit une endoprothèse tressée fabriquée en alliage à base de Cobalt-Nickel-Chrome dans laquelle, pour augmenter la résistance à la compression radiale, on a utilisé du fil doublé, ce qui pose un problème d'encombrement dans l'applicateur.

L'utilisation de tels fils pour faire des tresses médicales pourrait a priori donner de bons résultats. Néanmoins, la limite de résistance à la rupture du fil écroui se situe autour de 2000 N/mm² et après traitement thermique, le fil atteint des valeurs de résistance à la rupture de 2500 à 2700 N/mm², ce qui rend le fil rigide et cassant; ils se révèlent relativement difficiles à bobiner et à tresser du fait de leur élasticité propre. Des bris de fils fréquents abîment notamment les fuseaux des machines, qui sont soumises à une dégradation accélérée.

En outre, lors de l'utilisation à long terme, surtout pour les pathologies vasculaires où les sollicitations sur le métal sont très élevées (ex.: les anévrismes abdominaux), il s'est avéré que les stents réalisés à partir de ces fils vieillissaient rapidement (effets de fatigue).

Des essais de fatigue ont montré les mêmes résultats après une simulation en compression longitudinale pour une période équivalente de cinq mois.

D'autres endoprothèses autoexpansibles décrites, notamment dans EP-A-0 481 365, et DE-A-433.836 utilisent des alliages métalliques dotés d'une "mémoire de forme", par exemple des alliages Nickel-Titane, comme le Nitinol®.

Les armatures de ces endoprothèses sont fabriquées sous des formes variées (notamment des troncs de cylindre incisés, des structures hélicoïdales et des structures en mailles). Sous l'effet d'une activation (généralement thermique) elles tendent à adopter une forme radialement expansée qu'un traitement préalable les a forcées à "mémoriser".

De telles endoprothèses manquent généralement de flexibilité.

Ainsi, les troncs de cylindre incisés et les structures maillées sont rigides et plicaturent excessivement. En outre, ils occupent une place importante dans l'introducteur.

Les structures hélicoïdales (ou coils) n'ouvrent pas suffisamment les artères et sont inefficaces dans le traitement de sténoses, car elles ne couvrent pas tout le mur artériel.

Par ailleurs, aucun type de ces endoprothèses n'est utilisable dans des artères de gros calibre.

Il faut prévoir de surcroît la possibilité que la force de contrainte engendrée par la transition de phase des matériaux formant l'armature ne suffise pas pour vaincre la pression due à la paroi et les frictions. Dans ce cas, il y a un risque élevé que l'endoprothèse ne puisse pas se déployer.

L'opérateur doit prévoir la possibilité de l' introduction ultérieure d'un ballon gonflable pour amener l'endoprothèse à son diamètre nominal. Cette technique d'élargissement "forcé" entraîne fréquemment, à long terme, des réactions de l'organisme (notamment des proliférations de tissus).

L'invention a pour but la mise au point d'une endoprothèse présentant une bonne flexibilité durant sa phase de mise en place mais présentant sur site une bonne résistance à l'écrasement.

Un autre but de l'invention est que l'endoprothèse présente une bonne stabilité sur le site d'implantation.

Un autre but de l'invention est que la mise au point d'une endoprothèse qui couvre une large plage de diamètre et qui puisse notamment être implantée dans des conduits anatomiques de diamètre important.

L'invention a pour objet une endoprothèse luminale comprenant une armature en un matériau élastique, radialement expansible capable d'adopter une forme radialement contractée et une forme radialement étendue. Cette endoprothèse est caractérisée en ce que
- l'armature est formée de fils métalliques tressés
- le métal de l'armature a subi un traitement approprié lui imposant, dans une plage de température supérieure à la température ambiante et inférieure à la température d'un organisme à sang chaud, une transition d'état structurelle l'amenant d'une rigidité donnée à une rigidité supérieure.

Le métal est de préférence super-élastique, propriété lui permettant de se déformer de façon réversible de plusieurs pourcents. Les fils formant la tresse sont avantageusement en métal bio-compatible, et choisi parmi les alliages au Nickel-Titane et les alliages au Nickel-Titane-Cobalt. L'alliage comprend préférentiellement, entre 52 et 56% en poids de Nickel.

La tresse est formée de fils tressés qui se croisent sous un angle α lorsque l'armature est radialement étendue. Cet angle peut varier sur la longueur de la tresse. De préférence, ces fils forment un angle α compris entre 30 et 90° et, avantageusement, entre 50 et 70° sur au moins un tronçon de l'armature.

L'invention a également pour objet un procédé de fabrication pour une endoprothèse telle que décrite ci-dessus, qui comprend les opérations suivantes:
- confection de fils en un métal élastique auquel il est possible d'imposer une transition de structure atomique réversible le faisant passer d'une rigidité déterminée à une rigidité supérieure
- tressage de ces fils autour d'un mandrin de façon à obtenir une tresse
- soumission de tronçons de cette tresse, à leur diamètre nominal, a un traitement thermique établissant une transition de phase à une température égale ou inférieure à la température d'un organisme à sang chaud
- mise en place d'un revêtement éventuel sur ladite armature
- scission des tronçons de tresse en segments de longueur appropriée.

Avantageusement, le tressage est effectué avec des fils de Nickel-Titane écrouis bruts de filière et le traitement thermique comprend au moins une phase de chauffage dans une zone comprise entre 450 et 600°C avantageusement à 500°C pendant 10 minutes et un refroidissement à l'air.

Divers avantages de l'invention sont que l'endoprothèse permet un coefficient d'allongement très réduit, est très souple sous sa forme contractée, n'est pas sujette à plicature et résiste parfaitement à l'écrasement après sa mise en place.

Un autre avantage est qu'au-dessous de sa température de transition, l'endoprothèse est très facile à manipuler, de sorte qu'elle peut être facilement mise à dimension, amenée à son diamètre réduit et introduite dans un applicateur sans crainte de dégradation.

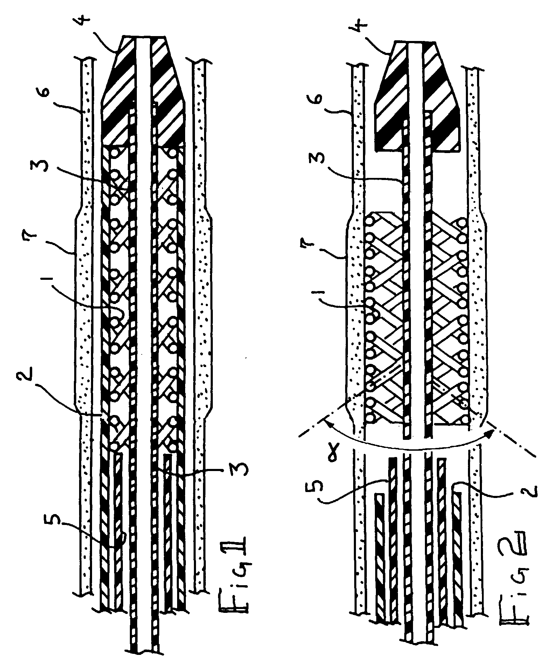

D'autres particularités et avantages de l'invention apparaîtront de la description de modes de réalisation particuliers, référence étant faite aux Figures annexées, dans lesquelles
les Figures 1 et 2 sont des représentations schématiques de deux étapes de mise en place d'une endoprothèse suivant l'invention dans un conduit anatomique.

La Figure 1 montre l'aspect général d'un dispositif de mise en place d'une endoprothèse suivant l'invention. Pour la clarté des dessins, seule l'armature 1 de l'endoprothèse y est représentée. Il est bien entendu que ce qui apparaît ici comme un simple stent peut comporter un revêtement extérieur et/ou intérieur (coating).

L'armature 1 est formée d'une tresse de fils métalliques entrelacés.

La particularité de l'endoprothèse suivant l'invention réside essentiellement dans la conception de son armature, qui fait intervenir les effets d'une structure tressée, naturellement élastique, et les propriétés physiques particulières des filaments qui la composent, liées à un effet de transition de phase.

Les fils formant l'armature tressée sont en effet réalisés à partir d'un alliage particulier (en l'occurrence, un alliage de Ni-Ti) qui, grâce à un traitement approprié décrit plus loin subit, à une température prédéterminée, proche de celle d'un organisme à sang chaud une transition de structure cristallographique réversible entraînant un changement radical de ses caractéristiques mécaniques.

Le métal de l'armature étant dans son état initial (c'est-à-dire, au-dessous de sa température de transition de phase) apparaît parfaitement ductile.

L'opérateur peut dans ces conditions manipuler très facilement l'endoprothèse, sans crainte de l'abîmer, de briser la structure ou de déranger l'agencement des filaments. Il peut notamment mettre l'endoprothèse à dimension en la coupant et la comprimer radialement (ce qui a pour effet de rapprocher les fils entre eux, leur angle de croisement tendant à ce moment vers une valeur négligeable proche de zéro).

L'endoprothèse étant dans cet état, l'opérateur peut aisément l'enfiler sur la tige creuse 3 d'un applicateur, entre une tête atraumatique 4 et un poussoir 5 et faire coulisser sur l'ensemble une gaine externe 2 qui maintient l'endoprothèse en place en la soumettant à une contrainte radiale, à ce stade presque négligeable.

La Figure 1 montre l'extrémité distale de l'applicateur après que celle-ci a été introduite par une incision ou par un orifice anatomique naturel dans un conduit anatomique 6, de façon à amener l'endoprothèse sur le site à traiter 7.

A l'instant où l'opérateur libère l'endoprothèse en faisant rétrocoulisser la gaine 2, la contrainte radiale cesse de s'exercer et, de par l'élasticité propre de la structure tressée, l'armature 1 se dilate jusqu'à son diamètre nominal, qui correspond sensiblement à celui du conduit anatomique 6.

Par ailleurs, la transition de structure cristallographique mentionnée plus haut se produit lorsque la température de l'armature atteint celle de l'organisme.

Ce changement sort ses effets au moment où l'armature 1 se déploie, entraînant un accroissement de la valeur de l'angle de croisement α entre les fils.

Les fils participent donc à double titre à l'ouverture de la structure tressée: il se produit un effet synergique important entre le déploiement de la structure en tresse de l'armature et la rigidification des fils due à leur transition d'état.

L'endoprothèse qui jusqu'au moment du largage faisait montre d'une très grande souplesse, parfaitement adaptée pour son insertion dans les sinuosités de conduits anatomiques se trouve donc rigidifiée et de façon quasi-instantanée parfaitement capable non seulement d'exercer une pression adéquate sur la paroi intérieure du conduit anatomique 6, mais aussi de résister aux sollicitations extérieures que devra subir ce conduit anatomique 6.

Les deux effets combinés (expansion mécanique couplée à une rigidification thermique) se renforcent l'un l'autre et permettent une expansion complète de l'endoprothèse sans traumatisme, ce qui est, à long terme, bénéfique pour le patient.

A performances équivalentes, le nombre des fils formant l'armature 1 de l'endoprothèse peut être réduit, ou, optionnellement on peut utiliser des fils de plus faible diamètre que dans les endoprothèses tressées suivant l'état de la technique, ce qui entraîne une réduction substantielle du diamètre de l'endoprothèse stent à l'état comprimé et donc de l'applicateur, et augmente la flexibilité.

Cette conception entraîne également d'autres avantages appréciables, notamment pour la mise en place dans les conduits anatomiques de gros diamètre.

Si l'on désire traiter des lésions très athéromateuses, ce n'est possible, avec des endoprothèses utilisant des armatures auto-expansibles classiques qu'au prix d'une technologie qu'on peut qualifier de "lourde" : ces armatures doivent alors être dotées d'éléments rigidifiants occupant une place importante (métal relativement épais, nombre de fils élevé et/ou de gros diamètre). Même sous leur forme radialement contractée, de telles endoprothèses présentent un diamètre et une longueur élevés.

En utilisant l'endoprothèse suivant l'invention, non seulement l'armature est allégée (d'où réduction du diamètre dans l'applicateur), mais de surcroît le risque de non-déploiement radial de l'endoprothèse est considérablement réduit.

La Figure 2 montre l'angle α formé par les fils tressés entre eux lorsque l'armature est déployée radialement.

L'endoprothèse donne en pratique d'excellents résultats, indépendamment de la valeur de l'angle choisie. Les essais ont permis de constater une excellente adaptation des endoprothèses suivant l'invention notamment pour des valeurs de α comprises entre 30 et 90° et, optimalement, dans une fourchette de 50 à 70°. Dans cette gamme d'angles, la différence de longueur entre l'endoprothèse comprimée (voir Figure 1) et relâchée (voir Figure 2) est proportionnellement faible.

On notera que le diamètre de l'endoprothèse (représentée ici comme quasi-cylindrique) peut varier sur sa longueur et qu'en conséquence l'angle α peut également varier suivant la section considérée de l'endoprothèse.

Un angle α de faible ouverture permet de réduire notablement les phénomènes de friction au moment du largage et de mieux adapter l'endoprothèse aux caractéristiques biomécaniques des conduits anatomiques.

Comme signalé plus haut, les phénomènes de friction, caractéristiques des endoprothèses présentant un coefficient d'allongement élevé, limitaient en effet les possibilités de traitement à des lésions de faible extension axiale.

De surcroît, les opérations de mise en place sont facilitées puisqu'on gagne en flexibilité, en précision de placement et en fiabilité de la manoeuvre de largage.

Même pour un faible angle de croisement α entre les fils, les essais cliniques révèlent un effet a priori paradoxal: l'endoprothèse n'a pas tendance à migrer suivant l'axe du conduit, comme le praticien pourrait s'y attendre.

La fabrication de l'armature d'endoprothèse suivant l'invention fait appel à un nombre réduit d'opérations, ce qui se répercute favorablement sur les coûts de production.

Même après traitement thermique déterminant la température à laquelle doit se produire la transition d'état, le présent fil "à mémoire de forme" reste souple et ne présente pas la rigidité d'un fil à mémoire conditionné classiquement : il atteint une résistance à la rupture d'à peine 1500 N/mm².

La fabrication de l'armature d'une endoprothèse suivant l'invention comprend généralement les opérations suivantes:
- confection de fils en alliage Nickel-Titane écroui
- tressage autour d'un mandrin de ces fils métalliques avant recuit à la sortie de la filière
- débitage de la tresse en tronçons
- soumission de tronçons de tresse, à leur diamètre nominal, à un traitement thermique établissant une transition de phase du métal à une température égale ou inférieure à la température de l'organisme.

La mémoire de forme a pour origine l'existence d'un changement cristallographique réversible qui se manifeste au cours de cycles de chauffage-refroidissement de l'échantillon. Dans le cas de métaux, la phase à haute température appelée austénite est caractérisée par une maille élémentaire de symétrie élevée (elle occupe un plus grand volume sans transfert de matière). La phase à basse température, dite martensite, a une maille de symétrie plus basse et occupe un volume minimum.

Cette phase peut apparaître sous plusieurs variantes équivalentes.

Afin d'obtenir le passage de l'un à l'autre de ces deux états d'une manière parfaitement reproductible, il faut parvenir à forcer la martensite dans une seule de ces variantes, de façon à obtenir la rigidité du métal adéquate venant en appoint à la structure physique de la tresse formant l'armature.

La composition de l'alliage (où le Nickel est présent dans une proportion variant entre 52 et 56% en poids) joue un rôle non négligeable pour la détermination des paramètres optimum.

On a constaté qu'une variation infime de la composition suffit à décaler de plusieurs degrés la température du traitement nécessaire pour obtenir une transformation martensitique donnée. Par exemple, une variation de 0,1% en poids de Nickel entraîne une variation de 15°C de la température du traitement thermique. Il convient donc de soigner tant la composition que les traitements lorsqu'on désire obtenir une température de transformation martensitique bien précise et des effets stables.

La structure tressée est préparée en la fixant sur une barre métallique d'un diamètre en rapport avec le diamètre nominal de l'endoprothèse ou en l'introduisant à l'intérieur d'un moule creux. L'ensemble subit un traitement thermique entre 400 et 600°C pendant une durée appropriée (généralement de l'ordre de 10 minutes), opération suivie d'un refroidissement à l'air. La température de transformation martensitique se situe alors entre 30 et 40°C. A titre d'exemple, pour un alliage à 55,7% de Nickel, écroui à 40%, on applique un temps de traitement de 10 minutes à 500° et le raidissement maximum est obtenu à 37°.

En fonction du diamètre des fils utilisés, le traitement thermique peut éventuellement être répété pour éliminer l'austénite résiduelle.

A titre de comparaison, le traitement thermique d'alliages ELGILOY® se fait sous vide à 550°C pendant +/- 4 heures.

Le tableau ci-après permet une comparaison de la résistance à la pression radiale de différentes armatures d'endoprothèses.

La méthode la plus connue et la plus facile se fait à l'aide d'un appareil extensomètre INSTRON®.

L'échantillon utilisé est porté à une température de 37°C, soit par bain thermostatique ou par air. On fait tourner autour de chaque armature un fil très fin d'environ 0,10 mm de diamètre. Une extrémité de ce fil est fixée à la base de l'appareil et l'autre est fixée à la partie supérieure de l'appareil qui est amovible.

Cette partie supérieure est constituée d'une sonde mesurant simultanément la force exercée sur l'échantillon et le déplacement.

La valeur résultante est déterminée (en gr) par un programme de calcul.

| Stent suivant l'invention (37°C) | | Fil en alliage de Cobalt | | Fil en alliage de Cobalt | |
|---|---|---|---|---|---|
| Valeur angle α | 32,5° | 35° | 45° | 55° | 53° |
| Longueur | 80 mm | 80 mm | 80 mm | 80 mm | 80 mm |
| Diamètre initial (mm) | 8,1 | 30,1 | 8,15 | 8,2 | 30,5 |

| Force (gr) | Diamètre sous contrainte (mm) | | | | |
|---|---|---|---|---|---|
| 0 | 8,1 | 30,1 | 8,15 | 8,2 | 30,5 |
| 20 | 8,1 | 30,1 | 7,5 | 7,98 | 30,5 |
| 40 | 8,1 | 30,1 | 7,2 | 7,91 | 30,2 |
| 60 | 8,1 | 30,1 | 8,95 | 7,3 | 29,8 |
| 80 | 8,1 | 30,1 | 6,3 | 6,98 | 26,1 |
| 100 | 8 | 29,9 | 5,5 | 6,2 | 23,54 |
| 120 | 7,99 | 29,6 | 4,2 | 5,7 | 20,05 |
| 140 | 7,98 | 29,3 | 3,1 | 4,9 | 19,4 |
| 160 | 7,97 | 29,1 | 2,5 | 3,9 | 16,36 |
| 180 | 7,96 | 28,9 | 2,1 | 3,2 | 12,6 |

On notera que les fils de Nickel-Titane utilisés se couvrent, suite au présent traitement thermique, d'une couche d'oxyde de titane qui assure la passivation du métal (à titre de comparaison, les surfaces métalliques de structures en alliage de cobalt doivent être passivées par un traitement ultérieur à l'acide nitrique ou phosphorique).

Les extrémités d'armatures en alliage classiques sont coupantes et agressives, du fait de la rigidité des fils. Les cas de perforation d'artères ou de la gaine de l'applicateur ne sont pas rares.

Les endoprothèses dotées d'une armature suivant l'invention sont au contraire non traumatiques et faciles à rogner, ce qui permet d'ajuster aisément leur longueur au cas par cas, au besoin sur le site même d'une intervention, à partir de segments de longueurs normalisées, ce qui facilite le conditionnement des éléments d'armature et des endoprothèses elles-mêmes.

## Revendications

1. Endoprothèse luminale comprenant une armature (1) radialement expansible, en un matériau élastique, capable d'adopter une forme radialement contractée et une forme radialement étendue
caractérisée en ce que
- l'armature est formée de fils métalliques tressés
- le métal formant les fils de l'armature (1) a subi un traitement approprié lui imposant, dans une plage de température supérieure à la température ambiante et inférieure à la température d'un organisme à sang chaud, une transition d'état structurelle l'amenant d'une rigidité donnée à une rigidité supérieure.

2. Endoprothèse suivant la revendication 1, caractérisée en ce que le métal des fils est superélastique.

3. Endoprothèse suivant la revendication 2, caractérisée en ce que le métal des fils est choisi parmi les alliages au Nickel-Titane et les alliages au Nickel-Titane-Cobalt.

4. Endoprothèse suivant la revendication 3, caractérisée en ce que l'alliage comprend entre 52 et 56% en poids de Nickel.

5. Endoprothèse suivant l'une quelconque des revendications 1 à 4, caractérisée en ce que l'angle α sous lequel les fils métalliques se croisent lorsque l'armature (1) est radialement étendue varie sur la longueur de la tresse.

6. Endoprothèse suivant l'une quelconque des revendications 1 à 5, caractérisée en ce que, sous sa forme radialement étendue, l'armature est formée de fils tressés formant entre eux, sur au moins un tronçon de la tresse, un angle α compris entre 30 et 90°.

7. Endoprothèse suivant la revendication 6, caractérisée en ce que l'angle α est compris entre 50 et 70°.

8. Procédé de fabrication pour une endoprothèse suivant l'une quelconque des revendications précédentes, caractérisé en ce qu'il comprend les opérations suivantes:
- confection de fils en un métal élastique auquel il est possible d'imposer une transition de structure atomique le faisant passer d'une rigidité déterminée à une rigidité supérieure
- tressage de ces fils autour d'un mandrin de façon à obtenir une tresse
- soumission de tronçons de cette tresse, à leur diamètre nominal, à un traitement thermique établissant une transition de phase à une température égale ou inférieure à la température d'un organisme à sang chaud
- mise en place d'un revêtement éventuel sur ladite armature
- scission des tronçons de tresse en segments de longueur appropriée.

9. Procédé suivant la revendication 8, caractérisée en ce que
- le tressage est effectué avec des fils de Nickel-Titane écrouis de filière, et en ce que
- le traitement thermique comprend au moins une opération de chauffage dans une zone comprise entre 400 et 600°C et un refroidissement à l'air.

10. Procédé suivant la revendication 9, caractérise en ce que le traitement thermique est mené à 500°C pendant 10 minutes.
